# EUROPÄISCHE PATENTANMELDUNG

(11) **EP 4 268 878 A2**
(43) Veröffentlichungstag der Anmeldung: **01.11.2023**
(21) Anmeldenummer: 23169811.9
(22) Anmeldetag: 25.04.2023
(51) Int. Cl.: A61M 25/02, F16L 3/00, H02G 1/00

(54) **VERANKERUNGSVORRICHTUNG ZUM VERANKERN EINER FLEXIBLEN LEITUNG SOWIE ENTSPRECHENDES SYSTEM**

(30) Priorität: 29.04.2022 DE 102022110565
(71) Anmelder: B. Braun Avitum AG, 34212 Melsungen (DE)
(72) Erfinder: KNIERIM, Michael, 34212 Melsungen-Schwarzenberg (DE)
(74) Vertreter: Winter, Brandl - Partnerschaft mbB

(57) **Zusammenfassung**

Verankerungsvorrichtung (2) zum Verankern einer flexiblen Leitung (64), mit einer Basis (6), die eine Vertiefung (60) aufweist, die ausgebildet ist, einen Teil der Leitung in sich aufnehmen zu können und die einen krummlinienförmigen Hohlraum definiert, der an einer ersten Stelle (66) in eine erste Richtung gekrümmt ist, um ein Herausgleiten des in der Vertiefung (60) aufgenommenen Teils der Leitung (64) in Erstreckungsrichtung der Vertiefung (60) in selbsthemmender Weise verhindern oder erschweren zu können, und einer Abdeckung (4), die in einer geöffneten Position die Vertiefung (60) für ein Einlegen der Leitung (64) freigibt und in einer geschlossenen Position die Vertiefung (60) abdeckt, um ein Herausgleiten des in der Vertiefung (60) aufgenommen Teils der Leitung (64) quer zur Erstreckungsrichtung der Vertiefung (60) verhindern oder erschweren zu können.

## Beschreibung

### Technisches Gebiet

Die vorliegende Offenbarung bezieht sich auf eine Verankerungsvorrichtung gemäß dem Oberbegriff des Anspruchs 1, insbesondere auf eine Verankerungsvorrichtung zum Verankern einer flexiblen Leitung, vorzugweise eines elektrischen Kabels und/oder eines zum Transport von Stoffen/Medien ausgebildeten Schlauchs, an einem medizinischen Gerät oder an einer zu behandelnden und/oder zu überwachenden Person. Außerdem bezieht sich die vorliegende Offenbarung auf ein System mit einer offenbarungsgemäßen Verankerungsvorrichtung sowie einer Leitung.

### Stand der Technik

Die EP 1 265 668 B1 offenbart eine Verankerungsvorrichtung zum Verankern einer flexiblen Leitung mit einer Basis, die eine Vertiefung aufweist, die einen krummlinienförmigen Hohlraum definiert, der an drei Stellen gekrümmt ist, um einen in der Vertiefung aufgenommenen Teil der Leitung ergreifen und ein Herausgleiten des in der Vertiefung aufgenommenen Teils der Leitung in Erstreckungsrichtung der Vertiefung in selbsthemmender Weise verhindern oder erschweren zu können. Des Weiteren weist die bekannte Verankerungsvorrichtung eine Abdeckung auf, die in einer geöffneten Position die Vertiefung für ein Einlegen der Leitung freigibt und in einer geschlossenen Position die Vertiefung abdeckt, um ein Herausgleiten des in der Vertiefung aufgenommen Teils der Leitung quer zur Erstreckungsrichtung der Vertiefung verhindern oder erschweren zu können.

Problem der Verankerungsvorrichtung nach EP 1 265 668 B1 ist, dass ein Einlegen einer Leitung in die Vertiefung aufgrund des krummlinienförmigen Verlaufs des Hohlraums aufwendig sein kann.

### Zusammenfassung der Offenbarung

Aufgabe der vorliegenden Erfindung ist es deshalb, eine Verankerungsvorrichtung bereitzustellen, die ausgebildet ist, eine Installation einer Leitung an der Verankerungsvorrichtung vereinfachen zu können, ohne zwangsläufig eine Verankerung der Leitung zu verschlechtern.

Diese Aufgabe wird durch eine Verankerungsvorrichtung mit den Merkmalen des Anspruchs 1 bzw. durch ein System mit den Merkmalen des Anspruchs 11 gelöst. Vorteilhafte Weiterbildungen sind Gegenstand der Unteransprüche.

Eine offenbarungsgemäße Verankerungsvorrichtung ist geeignet, eine flexible Leitung, insbesondere ein elektrisches Kabel und/oder einen zum Transport von Stoffen/Medien ausgebildeten Schlauch, insbesondere an einem medizinischen Gerät oder an einer zu behandelnden und/oder zu überwachenden Person, vorzugsweise wahlweise an einem medizinischen Gerät oder an einer zu behandelnden und/oder zu überwachenden Person, verankern zu können.

Die Verankerungsvorrichtung weist eine Basis und eine Abdeckung auf.

Die Basis weist eine Vertiefung auf, die ausgebildet ist, einen Teil der Leitung in sich aufnehmen zu können und die einen krummlinienförmigen Hohlraum definiert, der an einer ersten Stelle in eine erste Richtung gekrümmt ist, um ein Herausgleiten des in der Vertiefung aufgenommenen Teils der Leitung (aus der Vertiefung) in Erstreckungsrichtung der Vertiefung in selbsthemmender Weise verhindern oder erschweren zu können.

Die Vertiefung definiert den krummlinienförmigen Hohlraum insofern, als Flächen der Vertiefung derart geformt und/oder angeordnet sind, dass sie tangential zu Außenflächen des in der Vertiefung aufgenommenen Teils der Leitung sein und also in einem flächigen Kontakt mit dem in der Vertiefung aufgenommenen Teil der Leitung stehen können (definierter Umschlingungswinkel). Anders ausgedrückt, ist die Vertiefung derart ausgebildet, dass der in der Vertiefung aufgenommene Teil der Leitung aufgrund von flächigem Kontakt mit Flächen der Vertiefung, einen krummlinienförmigen Verlauf hat.

Die Abdeckung gibt in einer geöffneten Position (der Abdeckung relativ zur Basis) die Vertiefung für ein Einlegen der Leitung frei und deckt in einer geschlossenen Position (der Abdeckung relativ zur Basis) die Vertiefung ab, um ein Herausgleiten des in der Vertiefung aufgenommen Teils der Leitung (aus der Vertiefung) quer zur Erstreckungsrichtung der Vertiefung verhindern oder erschweren zu können.

Offenbarungsgemäß weist die Abdeckung einen Fassungsabschnitt auf, der in der geschlossenen Position der Abdeckung an einem Ende der Vertiefung angeordnet ist und der den von der Vertiefung definierten krummlinienförmigen Hohlraum derart verlängert, dass dieser im Bereich des Fassungsabschnitts an einer zweiten Stelle in eine quer oder entgegengesetzt zu der ersten Richtung verlaufende zweite Richtung gekrümmt ist.

Anders ausgedrückt, ist die Verankerungsvorrichtung derart ausgebildet, dass in der geöffneten Position der Abdeckung (relativ zur Basis) das Herausgleiten des in der Vertiefung aufgenommenen Teils der Leitung (aus der Vertiefung) in Erstreckungsrichtung der Vertiefung in selbsthemmender Weise, das heißt reibschlüssig, durch die Vertiefung verhindert oder erschwert werden kann und in der geschlossenen Position der Abdeckung (relativ zur Basis) das Herausgleiten des in der Vertiefung aufgenommenen Teils der Leitung (aus der Vertiefung) in Erstreckungsrichtung der Vertiefung in selbsthemmender Weise, das heißt reibschlüssig, sowohl durch die Vertiefung als auch durch den Fassungsabschnitt der Abdeckung verhindert oder erschwert werden kann und das Herausgleiten des in der Vertiefung aufgenommenen Teils der Leitung (aus der Vertiefung) quer zur Erstreckungsrichtung der Vertiefung formschlüssig durch die Abdeckung verhindert oder erschwert werden kann. Der Fassungsabschnitt ist also insbesondere ausgebildet, den durch die Vertiefung mittels einer ersten Krümmung verursachten (axialen) Reibschluss zwischen der Verankerungsvorrichtung und der Leitung mittels einer zweiten Krümmung zu sichern bzw. zu unterstützen.

Durch Vorsehen des Fassungsabschnitts an der Abdeckung und der funktionalen Aufteilung des Reibschlusses zwischen der Verankerungsvorrichtung und der Leitung einerseits auf einen Reibschluss zwischen der Basis und der Leitung und andererseits auf einen Reibschluss zwischen der Abdeckung und der Leitung ist es in vorteilhafter weise möglich, die Vertiefung derart einfach zu gestalten, dass ein Einlegen der Leitung leichtfällt, ohne die Verankerung der Leitung zu verschlechtern.

Gemäß einem Aspekt der Offenbarung kann die Abdeckung einen zweiten Fassungsabschnitt aufweisen, der in der geschlossenen Position der Abdeckung an einem zweiten Ende der Vertiefung angeordnet ist und der den von der Vertiefung definierten krummlinienförmigen Hohlraum derart verlängert, dass dieser im Bereich des zweiten Fassungsabschnitts an einer dritten Stelle in eine quer oder entgegengesetzt zu der ersten Richtung verlaufende dritte Richtung gekrümmt ist. Insbesondere können die beiden Fassungsabschnitte derart ausgebildet sein, dass eine von der Verankerungsvorrichtung aufgenommene Leitung an der zweiten Stelle und der dritten Stelle um dasselbe Maß gekrümmt wird.

Durch Vorsehen eines zweiten Fassungsabschnitts kann die reibschlüssige Wirkung der Abdeckung weiter verbessert werden. Bei der Verankerung eines Schlauches mithilfe des zweiten Fassungsabschnittes ist es des Weiteren möglich, eine resultierende Impulskraft, die von einem in dem Schlauch fließenden Fluid verursacht wird, in vorteilhafter Weise zu beeinflussen oder zumindest teilweise kompensieren zu können.

Gemäß einem Aspekt der Offenbarung können die zweite und die dritte Richtung parallel zueinander sein bzw. verlaufen.

Werden die beiden Fassungsabschnitte derart ausgebildet, dass eine von der Verankerungsvorrichtung aufgenommene Leitung an der zweiten Stelle und der dritten Stelle in zueinander parallele Richtungen gekrümmt wird, ist es möglich, ein Schließen der Abdeckung (d.h. ein Bewegen der Abdeckung von der geöffneten Position in die geschlossene Position) ergonomisch vorteilhaft gestalten zu können. Dieser kann insbesondere erreicht werden, wenn die von der Verankerungsvorrichtung aufgenommene Leitung an der zweiten Stelle und der dritten Stelle in zueinander parallele Richtungen um dasselbe Maß gekrümmt wird.

Gemäß einem Aspekt der Offenbarung kann die Basis eine Grundplatte und drei oder mehr sich von der Grundplatte aus erstreckende Stifte aufweisen und kann die Vertiefung durch zwei oder mehr sich zwischen den Stiften erstreckende Zwischenräume gebildet sein.

Als "Stift" wird in diesem Zusammenhang eine längliche Struktur verstanden, deren Längserstreckung größer ist als deren mittlere Quererstreckung.

Zumindest einer der Stifte kann insbesondere derart ausgebildet sein, dass dessen freies Ende eine größere Quererstreckung hat als angrenzende Bereiche des Stiftes, so dass die Vertiefung eine Hinterschneidung aufweist. Vorzugsweise können zwei der drei Stifte oder alle drei Stifte derart ausgebildet sein, dass das jeweilige freie Ende eine größere Quererstreckung hat als angrenzende Bereiche des entsprechenden Stiftes.

Indem die Vertiefung mittels Stifte gebildet wird, kann die Vertiefung elastisch ausgebildet werden, ohne verschiedene Materialien vorsehen zu müssen. Werden Stifte mit verbreiterten freien Enden vorgesehen, ist es möglich, mit einer in der Vertiefung aufgenommenen Leitung eine Clipverbindung herzustellen.

Gemäß einem Aspekt der Offenbarung kann die Abdeckung derart ausgebildet sein, dass sich in der geschlossenen Position der Abdeckung zumindest ein Teil der Abdeckung zumindest an einen der Stifte entlang seiner Längserstreckung anlegt.

"Anlegen" heißt in diesem Zusammenhang, dass ein flächiger Kontakt hergestellt wird.

Gemäß einem Aspekt der Offenbarung kann die Abdeckung zumindest einen der drei Stifte der Grundplatte enthalten, bspw. den Mittleren. In einer weiteren Ausführungsform kann die Abdeckung bzw. ein Fassungsabschnitt eines Fassungsteils die Aufgabe eines Stiftes übernehmen, um auf diese Weise den krummlinigen Hohlraum mit den weiteren Stiften der sich von der Grundplatte erstreckenden Stiften zu definieren/festzulegen.

Insbesondere kann die Abdeckung derart ausgebildet sein, dass sie zumindest einen Stift aufweist, der sich in der geschlossenen Position der Abdeckung an einen der Stifte der Basis anlegt. Vorzugsweise kann die Abdeckung zwei Stifte aufweisen, die sich in der geschlossenen Position der Abdeckung jeweils an einen entsprechenden Stift der drei Stifte der Basis anlegen. Insbesondere können die zwei Stifte der Abdeckung derart ausgebildet sein, dass sich in der geschlossenen Position der Abdeckung an die beiden außenliegenden Stifte der Basis anlegen.

Wird die Abdeckung derart ausgebildet, dass Teile von ihr in der geschlossenen Position der Abdeckung die Stifte der Basis flächig kontaktieren, ist es in vorteilhafter Weise möglich, die Steifigkeit der Vertiefung in der geöffneten Position der Abdeckung für ein einfaches Einlegen der Leitung gering halten zu können und die Steifigkeit der Vertiefung in der geschlossenen Position der Abdeckung für ein Halten der eingelegten Leitung im Vergleich zu der geöffneten Position zu erhöhen.

Gemäß einem Aspekt der Offenbarung kann die Abdeckung über ein Abdeckungsscharnier schwenkbar mit der Basis verbunden sein und kann das Abdeckungsscharnier an dem Fassungsabschnitt, insbesondere auch an dem zweiten Fassungsabschnitt, ausgebildet sein.

Wird der Fassungsabschnitt, der für zumindest eine weitere Krümmung einer in der Vertiefung aufgenommenen Leitung sorgen kann, bzw. werden die Fassungsabschnitte in unmittelbarer Nähe des Abdeckungsscharniers angeordnet, ist es möglich, die Leitung in besonders ergonomischer Weise krümmen zu lassen.

Gemäß einem Aspekt der Offenbarung kann die Abdeckung einen Kopplungsabschnitt aufweisen, der über ein Kopplungsabschnittsscharnier schwenkbar mit dem übrigen Teil der Abdeckung verbunden ist und der ausgebildet ist, in der geschlossenen Position der Abdeckung werkzeuglos lösbar mit der Basis gekoppelt werden zu können und das Herausgleiten des in der Vertiefung aufgenommen Teils der Leitung quer zur Erstreckungsrichtung der Vertiefung verhindern oder erschweren zu können. Insbesondere kann der Kopplungsabschnitt derart ausgebildet sein, dass der Fassungsabschnitt bzw. die Fassungsabschnitte abseits des Kopplungsabschnittes angeordnet ist bzw. sind. Vorzugsweise kann die Abdeckung derart ausgebildet sein, dass ein Stift oder mehrere Stifte an dem Kopplungsabschnitt ausgebildet sind. Der Stift oder die Stifte können insbesondere derart ausgebildet sein, dass der Stift bzw. die Stifte in einer gekoppelten Position des Kopplungsabschnitts (relativ zur Basis) formschlüssig und/reibschlüssig mit der Basis bzw. mit einem Teil der Basis zusammenwirken kann bzw. können. Vorzugsweise kann der Stift derselbe Stift sein, der an den entsprechenden Stift an der Basis angelegt werden kann bzw. können die Stifte dieselben Stifte sein, die an die entsprechenden Stifte an der Basis angelegt werden können, indem die Abdeckung in die geschlossene Position gebracht wird. Vorzugsweise kann die Basis einen Clip bzw. eine Federzunge aufweisen und kann der Kopplungsabschnitt der Abdeckung derart ausgebildet, in der gekoppelten Position des Kopplungsabschnitts (relativ zur Basis) formschlüssig mit dem Clip bzw. der Federzunge zusammen zu wirken. Die Federzunge kann auch an der Abdeckung angeordnet sein.

Durch die Ausbildung des schwenkbaren Kopplungsabschnitts kann die Bedienbarkeit der Verankerungsvorrichtung in vorteilhafterweise verbessert werden.

Allgemein wäre die Funktion auch mit einem Scharnier, statt zwei Scharnieren möglich. Der Vorteil von zwei Scharnieren ist die bessere Anbindungsmöglichkeit an der Basis. Des Weiteren wird das Material in den Filmscharnieren weniger belastet, da nur eine Verformung von 90 Grad stattfindet (bei einem Scharnier wären es 180 Grad).

Gemäß einem Aspekt der Offenbarung kann eine Schwenkachse des Kopplungsabschnittsscharniers, bezüglich welcher der Kopplungsabschnitt relativ zu dem übrigen Teil der Abdeckung schwenkbar ist, parallel zu einer Schwenkachse des Abdeckungsscharniers sein, bezüglich welcher die Abdeckung relativ zur Basis schwenkbar ist.

Indem die Schwenkachse des Kopplungsabschnittsscharniers parallel zu der Schwenkachse des Abdeckungsscharniers ausgerichtet wird, kann die Bedienbarkeit der Verankerungsvorrichtung weiter verbessert werden.

Gemäß einem Aspekt der Offenbarung können die Basis sowie die Abdeckung integral ausgebildet sein und können das Abdeckungsscharnier sowie das Kopplungsabschnittsscharnier als Filmscharniere ausgebildet sein.

Wird die Verankerungsvorrichtung integral ausgebildet, kann der Aufwand für die Herstellung der Verankerungsvorrichtung hinsichtlich Kosten und/oder Zeit reduziert werden.

Gemäß einem Aspekt der Offenbarung kann die Basis eine Sensoraufnahme aufweisen. Insbesondere kann die Sensoraufnahme als eine zylinderförmige, insbesondere kreiszylinderförmige, Ausnehmung ausbildet sein.

Wird die Verankerungsvorrichtung mit der Sensoraufnahme ausgebildet, ist es möglich, einen Sensor zusammen mit einer zugentlasteten Leitung an einem medizinischen Gerät oder an einer zu behandelnden und/oder zu überwachenden Person effizient zu befestigen.

Die Offenbarung betrifft des Weiteren ein System mit einer offenbarungsgemäßen Verankerungsvorrichtung und einer entsprechenden Leitung. Insbesondere kann die Verankerungsvorrichtung eine Sensoraufnahme aufweisen. Vorzugsweise kann das System einen Sensor aufweisen.

### Kurzbeschreibung der Zeichnungen

Die vorliegende Erfindung wird im Folgenden anhand eines bevorzugten Ausführungsbeispiels unter Bezugnahme auf die beigefügten Zeichnungen näher beschrieben. Es zeigen:
Fig. 1 eine perspektivische Ansicht einer offenbarungsgemäßen Verankerungsvorrichtung in einem geöffneten Zustand, in welchem eine Abdeckung relativ zu einer Basis in einer geöffneten Position ist;
Fig. 2 eine perspektivische Ansicht eines offenbarungsgemäßen Systems in einem Zustand, in welchem eine an einer ersten Stelle gekrümmte Leitung in die geöffnete Verankerungsvorrichtung gemäß Fig. 1 eingelegt ist;
Fig. 3 eine perspektivische Ansicht des in Fig. 2 gezeigten Systems in einem Zustand, in welchem die in der Verankerungsvorrichtung eingelegte Leitung zusätzlich an einer zweiten Stelle und an einer dritten Stelle gekrümmt ist;
Fig. 4 eine perspektivische Ansicht des in Fig. 3 gezeigten Systems in einem Zustand, in welchem die Abdeckung der Verankerungsvorrichtung aus der geöffneten Position in Richtung einer geschlossenen Position verschwenkt ist; und
Fig. 5 eine perspektivische Ansicht des in Fig. 4 gezeigten Systems in einem Zustand, in welchem die Abdeckung der Verankerungsvorrichtung in der geschlossenen Position ist.

### Detaillierte Beschreibung bevorzugter Ausführungsformen

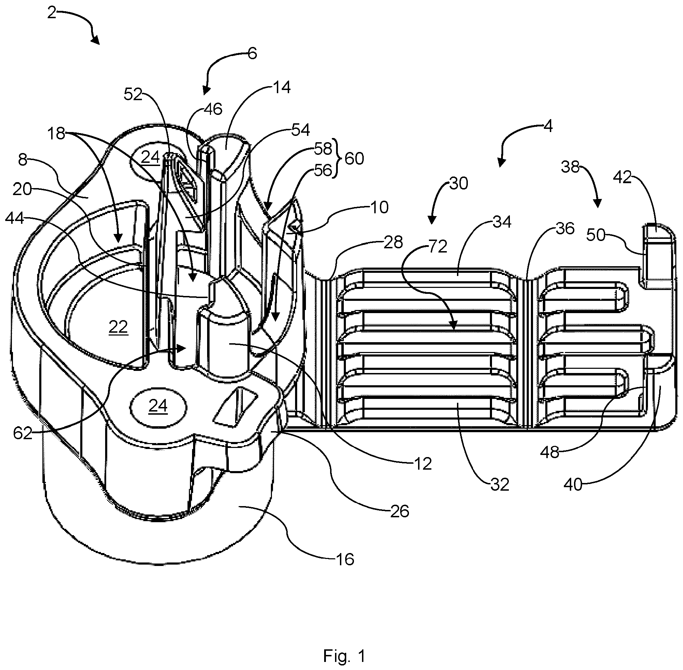

Fig. 1 zeigt eine perspektivische Ansicht einer offenbarungsgemäßen Verankerungsvorrichtung 2 in einem geöffneten Zustand, in welchem eine Abdeckung 4 relativ zu einer Basis 6 in einer geöffneten Position ist.

Die Basis 6 weist eine Grundplatte 8 auf, von dessen Oberseite sich drei Stifte 10, 12 und 14 nach oben erstrecken. Die Grundplatte 8 hat den Umriss einer abgerundeten Raute. Die drei Stifte 10, 12 und 14 sind in einem Dreieck angeordnet. Der mittlere Stift 10 ist an einer Ecke der rautenförmigen Grundplatte 8 angeordnet und die beiden anderen bzw. äußeren Stifte 12 und 14 sind an den beiden an der Ecke des mittleren Stifts 10 angrenzenden Seiten der rautenförmigen Grundplatte 8 angeordnet.

Von der Unterseite der Grundplatte 8 aus erstreckt sich ein hohlzylinderförmiger Vorsprung 16 nach unten, der konzentrisch mit der Grundplatte 8 ausgebildet ist. In der Grundplatte 8 sind zwei halbkreiszylinderförmige Ausnehmungen 18 ausgebildet, deren ebene Seiten derart parallel zueinander angeordnet sind, dass deren runde Seiten in einer gemeinsamen Kreiszylindermantelfläche liegen. Zwischen den Ausnehmungen 18 ist in der Grundplatte 8 eine Strebe 20 ausgebildet. Die gemeinsame Kreiszylindermantelfläche der Ausnehmungen 18 ist konzentrisch zu einem von dem hohlzylinderförmigen Vorsprung 16 definierten, kreiszylinderförmigen Raum. Die beiden Ausnehmungen 18 durchdringen die Grundplatte 8, so dass die Ausnehmungen 18 mit dem von dem hohlzylinderförmigen Vorsprung 16 definierten, kreiszylinderförmigen Raum verbunden sind. Der von dem hohlzylinderförmigen Vorsprung 16 definierte, kreiszylinderförmige Raum bildet eine Sensoraufnahme 22, deren Oberseite aufgrund der Ausnehmungen 18 teilweise von oben zugänglich ist. Die Strebe 20 bildet einen Anschlag für einen in der Sensoraufnahme 22 aufgenommenen (nicht gezeigten) Sensor und/oder sorgt für eine Versteifung (Festigkeitserhöhung) der Basis 6 bzw. der Grundplatte 8.

An den beiden spitzwinkligen Ecken der rautenförmigen Grundplatte 8 ist jeweils eine senkrecht zur Erstreckungsebene der Grundplatte 8 ausgerichtete bzw. parallel zu dem Vorsprung 16 verlaufende, kreiszylinderförmige Befestigungsausnehmung 24 ausgebildet. Die Befestigungsausnehmungen 24 sind dazu ausgebildet, die Verankerungsvorrichtung 2 beispielsweise mittels (nicht gezeigter) Schrauben oder Clips an ein Gehäuse eines medizinischen Geräts oder an einem Fixierverband einer zu behandelten bzw. überwachenden Person befestigen zu können.

An einer der beiden spitzwinkligen Ecken der rautenförmigen Grundplatte 8 ist ein bogenförmiger Vorsprung 26 vorgesehen, welcher derart ausgebildet ist, dass ein Kennzeichnungsband oder eine Sicherheitsplombe für die Verankerungsvorrichtung 2 an dem Vorsprung 26 befestigt werden kann. Alternativ oder zusätzlich kann der bogenförmige Vorsprung 26 derart angeordnet und/oder ausgebildet, dass an dem bogenförmigen Vorsprung 26 mit einem Kabelbinder oder ähnliches ein (neuer bzw. weiterer) Verankerungspunkt für die Leitung 64 an der Verankerungsvorrichtung 2 und/oder ein Verankerungspunkt für die Verankerungsvorrichtung 2 an einem medizinischen Gerät erzeugt werden kann.

An derjenigen stumpfwinkligen Ecke der rautenförmigen Grundplatte 8, an welcher der mittlere Stift 12 angeordnet ist, ist an einem unteren Rand der Grundplatte 8 ein Abdeckungsscharnier 28 ausgebildet.

Die Abdeckung 4 ist über das Abdeckungsscharnier 28 schwenkbar mit der Basis 6 bzw. der Grundplatte 8 verbunden.

Die Abdeckung 4 weist einen Fassungsteil 30 mit einem Fassungsabschnitt 32 und einem zweiten Fassungsabschnitt 34 auf. Der Fassungsteil 30 ist mit dem Abdeckungsscharnier 28 verbunden. Über ein Kopplungsabschnittscharnier 36 ist ein Kopplungsabschnitt 38 schwenkbar mit dem Fassungsteil 30 verbunden.

Die Abdeckung 4 ist bandförmig ausgebildet, so dass der Fassungsteil 30 im Wesentlichen einer rechteckigen Platte entspricht, an deren beiden kurzen Seiten zum einen das Abdeckungsscharnier 28 und zum anderen das Kopplungsabschnittsscharnier 36 angeordnet sind.

Der Kopplungsabschnitt 38 entspricht ebenfalls im Wesentlichen einer rechteckigen Platte, die an einer Seite über das Kopplungsabschnittscharnier 36 mit dem Fassungsteil 30 verbunden ist. An den beiden freien Ecken des rechteckplattenförmigen Kopplungsabschnitt 38 sind Kopplungsstifte 40 und 42 ausgebildet, die senkrecht zu einer Erstreckungsebene des Kopplungsabschnitts 38, in der geöffneten Position der Abdeckung 4 nach oben ausgerichtet sind. Die Kopplungsstifte 40 und 42 sind ausgebildet, die Abdeckung 4 im geschlossenen Zustand in Position zu halten und ein Verrutschen der Abdeckung 4 relativ zur Basis 6 bzw. der Grundplatte 8 zu verhindern bzw. zu erschweren. Nach oben kann die sich Abdeckung 4 aufgrund einer Federzunge 52 bzw. eines Schnapphaken nicht lösen.

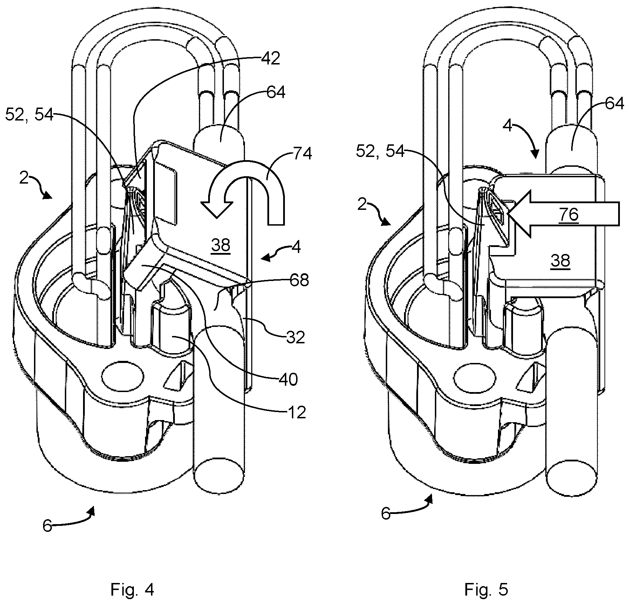

Die äußeren Stifte 12 und 14 der Basis 6 sind derart ausgebildet, dass sie jeweils eine Außenfläche bzw. Kontaktfläche 44 bzw. 46 aufweisen, die mit einer entsprechenden Außenfläche bzw. Kontaktfläche 48 bzw. 50 des entsprechenden Kopplungsstiftes 40 bzw. 42 in flächige Anlage kommen können, wenn die Abdeckung 4 relativ zu der Basis 6 in einer geschlossenen Position ist (siehe Fig. 5).

Von der Strebe 20 der Grundplatte 8 aus erstreckt sich die Federzunge 52 nach oben. Die Federzunge 52 ist im Wesentlichen parallel zu den Stiften 10, 12 und 14 ausgerichtet. Die Federzunge 52 und die Stifte 10, 12 und 14 sind derart in einer Raute angeordnet, dass die Federzunge 52 und der mittlere Stift 10 an den stumpfwinkligen Ecken der Raute angeordnet sind und die beiden äußeren Stifte 12 und 14 an den spitzwinkligen Ecken der Raute angeordnet sind. An dem freien Ende weist die Federzunge 52 einen keilförmigen Haken 54 auf.

Zwischen dem mittleren Stift 10 und dem äußeren Stift 12 bzw. 14 ist die Grundplatte 8 bogenförmig ausgebildet, so dass ein Zwischenraum 56 zwischen dem mittleren Stift 10 und dem äußeren Stift 12 und ein Zwischenraum 58 zwischen dem mittleren Stift 10 und dem äußeren Stift 14 zusammen eine zumindest abschnittsweise rinnenförmige Vertiefung 60 definieren. Aufgrund der Anordnung der Stifte 10, 12 und 14 in einem Dreieck ist die zumindest abschnittsweise rinnenförmige Vertiefung 60 krummlinienförmig bzw. weist die zumindest abschnittsweise rinnenförmige Vertiefung 60 einen Knick auf. Die Federzunge 52 ist breiter ausgebildet als die Stifte 10, 12 und/oder 14, so dass Zwischenräume 62 zwischen der Federzunge 52 und dem jeweiligen äußeren Stift 12 bzw. 14 kleiner sind als der Zwischenraum 56 bzw. 58.

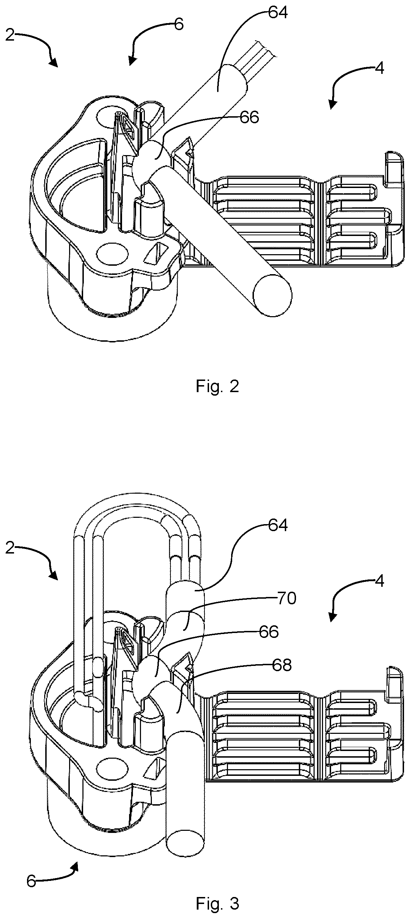

Fig. 2 zeigt eine perspektivische Ansicht eines offenbarungsgemäßen Systems in einem Zustand, in welchem die Verankerungsvorrichtung 2 geöffnet bzw. die Abdeckung 4 relativ zur Basis 6 in der geöffneten Position ist und eine Leitung 64 in die Vertiefung 60 eingelegt ist.

Aufgrund der dreieckförmigen Anordnung der Stifte 10, 12 und 14 bzw. der krummlinienförmigen Ausbildung der Vertiefung 60 ist die eingelegte Leitung 64 an einer ersten Stelle 66 bzw. in einem ersten Abschnitt gekrümmt. Diese Krümmung gewährleistet bereits eine gewisse Selbsthemmung, aufgrund derer ein Herausgleiten der Leitung 64 aus der Vertiefung 60 in Erstreckungsrichtung der Vertiefung 60 verhindert oder erschwert wird.

In dem in Fig. 2 gezeigten Zustand verläuft die Leitung 64 ausgehend von den beiden Zwischenräumen 56 und 58 bzw. von den beiden Enden der Vertiefung 60 in der Flucht der beiden Zwischenräume 56 und 58.

In dem in Fig. 3 gezeigten Zustand ist die Leitung 64 in einem Bereich angrenzend an den Zwischenraum 56 an einer zweiten Stelle 68 bzw. in einem zweiten Abschnitt sowie in einem Bereich angrenzend an den Zwischenraum 58 an einer dritten Stelle 70 bzw. in einem dritten Abschnitt gekrümmt. Dadurch, dass die Leitung 64 nicht nur an der ersten Stelle 66, sondern zusätzlich auch an der zweiten Stelle 68 und der dritten Stelle 70 gekrümmt ist, kann die durch die lediglich an der ersten Stelle 66 hervorgerufene Selbsthemmung unterstützt werden. Die Krümmungen an der zweiten Stelle 68 und der dritten Stelle 70 sind der Krümmung an der ersten Stelle 66 entgegengesetzt und betragsmäßig derart, dass die an der zweiten Stelle 68 und der dritten Stelle 70 angrenzenden, aus der Verankerungsvorrichtung 2 ragenden Abschnitte der Leitung 64 kollinear verlaufen. In dem in Fig.3 gezeigten Zustand ist die Leitung 64 bereits an den drei Stellen 66, 68 und 70 gekrümmt. Die Verankerungsvorrichtung 2 ist insbesondere derart ausgebildet, dass die Krümmungen an der zweiten und dritten Stelle 68 bzw. 70 durch ein Schließen der Abdeckung 4 hervorgerufen werden kann.

Um die Leitung 64 an der zweiten Stelle 68 und der dritten Stelle 70 zu krümmen, sind die Fassungsabschnitte 32 und 34 an dem Fassungsteil 30 der Abdeckung 4 vorgesehen.

Fig. 4 zeigt eine perspektivische Ansicht des in Fig. 3 gezeigten Systems in einem Zustand, in welchem die Abdeckung 4 der Verankerungsvorrichtung 2 aus der geöffneten Position in Richtung der geschlossenen Position verschwenkt ist. In dem in Fig. 4 gezeigten Zustand ist der Fassungsteil 30 derart an die Grundplatte 8 geschwenkt, dass der Fassungsabschnitt 32 die Krümmung der Leitung 64 an der zweiten Stelle 68 gewährleistet, der zweite Fassungsabschnitt 34 die Krümmung der Leitung 64 an der dritten Stelle 70 gewährleistet, sich ein Abschnitt 72 des Fassungsteils 30, welcher zwischen dem Fassungsabschnitt 32 und dem zweiten Fassungsabschnitt 34 angeordnet ist (siehe Fig. 1), an einer äußeren Seite bzw. Fläche des mittleren Stiftes 10 anliegt und das dem Kopplungsabschnittscharnier 36 gegenüberliegende freie Ende des Kopplungsabschnitts 38 auf dem keilförmigen Haken 54 der Federzunge 52 aufliegt.

Wird die Abdeckung 4 bzw. der Kopplungsabschnitt 38 aus der in Fig. 4 gezeigten Position weiter in Richtung der Basis 6 verschwenkt (siehe Pfeil 74 in Fig.4), bewegen sich die Kopplungsstifte 40 und 42 auf die entsprechenden Stifte 12 und 14 der Basis 6 zu und hintergreifen die Stifte 12 und 14 derart, dass ein Verschwenken des Fassungsteils 34 weg von der Basis 6 durch Zusammenwirken der Kopplungsstifte 40 und 42 mit den Stiften 12 und 14 der Basis 6 bzw. durch ein Zusammenwirken der Kontaktflächen 48, 50 auf Seiten der Abdeckung 4 mit den entsprechenden Kontaktflächen 44 und 46 auf Seiten der Basis 6 verhindert wird. Während des Zuschwenkens des Kopplungsabschnitts 38 und des dabei auftretenden Hintergreifens der Stifte 12 und 14 durch die Kopplungsstifte 40 und 42 bewirkt ein Zusammenwirken des freien Endes des Kopplungsabschnitts 38 mit einer schrägen Fläche des keilförmigen Hakens 54 ein Ausweichen der Federzunge 52 in Richtung weg von dem mittleren Stift 10. Wenn der Kopplungsabschnitt 38 derart verschwenkt ist, dass die Kopplungsstifte 40 und 42 und die Stifte 12 und 14 zueinander parallel ausgerichtet sind und die Kontaktflächen 48 und 50 der Kopplungsstifte 40 und 42 auf den Kontaktflächen 44 und 46 der Stifte 12 und 14 aufliegen, schnappt die Federzunge 52 derart zu, dass der keilförmige Haken 54 das freie Ende des Kopplungsabschnitts 38 formschlüssig um- bzw. hintergreift. In dieser Position des Kopplungsabschnitts 38 befindet sich die Abdeckung 4 in der geschlossenen Position, welche in Fig. 5 gezeigt ist.

In dem in Fig. 5 gezeigten Zustand wird ein Herausgleiten der Leitung 64 aus der Vertiefung 60 in Richtung quer zur Erstreckungsrichtung der Vertiefung 60 durch den Kopplungsabschnitt 38 verhindert bzw. erschwert und in Richtung der Erstreckungsrichtung der Vertiefung 60 durch die von der Vertiefung 60 sowie von den Fassungsabschnitten 32 und 34 hervorgerufene Selbsthemmung verhindert bzw. erschwert.

Um die Leitung 64 der Verankerungsvorrichtung 2 zu entnehmen, muss die Federzunge 52 von dem Kopplungsabschnitt 38 derart weggeschwenkt werden (siehe Pfeil 76 in Fig. 5), dass der Haken 54 nicht mehr mit dem freien Ende des Kopplungsabschnitt 38 formschlüssig zusammenwirkt und der Kopplungsabschnitt 38 von der Basis 6 weggeschwenkt werden kann.

Die Verankerungsvorrichtung 2 gemäß den Figuren 1 bis 5 ist einheitlich aus einem thermoplastischen Kunststoff hergestellt.

### Bezugszeichenliste

- 2: Verankerungsvorrichtung
- 4: Abdeckung
- 6: Basis
- 8: Grundplatte
- 10: mittlerer Stift
- 12, 14: äußerer Stift
- 16: hohlzylinderförmiger Vorsrpung
- 18: halbkreiszylinderförmige Ausnehmung
- 20: Strebe
- 22: Sensoraufnahme
- 24: Befestigungsausnehmung
- 26: bogenförmiger Vorsprung
- 28: Abdeckungsscharnier
- 30: Fassungsteil
- 32: (erster) Fassungsabschnitt
- 34: zweiter Fassungsabschnitt
- 36: Kopplungsabschnittscharnier
- 38: Kopplungsabschnitt
- 40, 42: Kopplungsstift
- 44, 46: Kontaktfläche an dem Stift
- 48, 50: Kontaktfläche an dem Kopplungsstift
- 52: Federzunge
- 54: Haken
- 56, 58: Zwischenraum zwischen Stiften
- 60: Vertiefung
- 62: Zwischenraum zwischen Federzunge und Stift
- 64: Leitung
- 66: erste Stelle
- 68: zweite Stelle
- 70: dritte Stelle
- 72: Abschnitt am Fassungsteil zwischen den Fassungsabschnitten
- 74: Pfeil, der ein Zuschwenken des Kopplungsabschnitts anzeigt
- 76: Pfeil, der ein Öffnen der Federzunge anzeigt

## Patentansprüche

1. Verankerungsvorrichtung (2) zum Verankern einer flexiblen Leitung (64), mit
einer Basis (6), die eine Vertiefung (60) aufweist, die ausgebildet ist, einen Teil der Leitung in sich aufnehmen zu können und die einen krummlinienförmigen Hohlraum definiert, der an einer ersten Stelle (66) in eine erste Richtung gekrümmt ist, um ein Herausgleiten des in der Vertiefung (60) aufgenommenen Teils der Leitung (64) in Erstreckungsrichtung der Vertiefung (60) in selbsthemmender Weise verhindern oder erschweren zu können, und
einer Abdeckung (4), die in einer geöffneten Position die Vertiefung (60) für ein Einlegen der Leitung (64) freigibt und in einer geschlossenen Position die Vertiefung (60) abdeckt, um ein Herausgleiten des in der Vertiefung (60) aufgenommen Teils der Leitung (64) quer zur Erstreckungsrichtung der Vertiefung (60) verhindern oder erschweren zu können,
**dadurch gekennzeichnet, dass**
die Abdeckung (4) einen Fassungsabschnitt (32) aufweist, der in der geschlossenen Position der Abdeckung (4) an einem Ende der Vertiefung (60) angeordnet ist und der den von der Vertiefung (60) definierten krummlinienförmigen Hohlraum derart verlängert, dass dieser im Bereich des Fassungsabschnitts (32) an einer zweiten Stelle (68) in eine quer oder entgegengesetzt zu der ersten Richtung verlaufende zweite Richtung gekrümmt ist.

2. Verankerungsvorrichtung (2) nach Anspruch 1, **dadurch gekennzeichnet, dass** die Abdeckung (4) einen zweiten Fassungsabschnitt (34) aufweist, der in der geschlossenen Position der Abdeckung (4) an einem zweiten Ende der Vertiefung (60) angeordnet ist und der den von der Vertiefung (60) definierten krummlinienförmigen Hohlraum derart verlängert, dass dieser im Bereich des zweiten Fassungsabschnitts (34) an einer dritten Stelle (70) in eine quer oder entgegengesetzt zu der ersten Richtung verlaufende dritte Richtung gekrümmt ist.

3. Verankerungsvorrichtung (2) nach Anspruch 2, **dadurch gekennzeichnet, dass** die zweite und die dritte Richtung parallel zueinander verlaufen.

4. Verankerungsvorrichtung (2) nach einem der Ansprüche 1 bis 3, **dadurch gekennzeichnet, dass** die Basis (6) eine Grundplatte (8) und drei sich von der Grundplatte aus erstreckende Stifte (12, 14, 16) aufweist und die Vertiefung (60) durch zwei sich zwischen den Stiften (12, 14, 16) erstreckende Zwischenräume (56, 58) gebildet ist.

5. Verankerungsvorrichtung (2) nach Anspruch 4, **dadurch gekennzeichnet, dass** die Abdeckung (4) derart ausgebildet ist, dass sich in der geschlossenen Position der Abdeckung (4) zumindest ein Teil (72) der Abdeckung (4) zumindest an einen der Stifte (10) entlang seiner Längserstreckung anlegt.

6. Verankerungsvorrichtung (2) nach einem der Ansprüche 1 bis 5, **dadurch gekennzeichnet, dass** die Abdeckung (4) über ein Abdeckungsscharnier (28) schwenkbar mit der Basis (6) verbunden ist und das Abdeckungsscharnier (28) an dem Fassungsabschnitt (32; 34) ausgebildet ist.

7. Verankerungsvorrichtung (2) nach einem der Ansprüche 1 bis 6, **dadurch gekennzeichnet, dass** die Abdeckung (4) einen Kopplungsabschnitt (38) aufweist, der über ein Kopplungsabschnittsscharnier (36) schwenkbar mit dem übrigen Teil (30) der Abdeckung (4) verbunden ist und der ausgebildet ist, in der geschlossenen Position der Abdeckung (4) werkzeuglos lösbar mit der Basis (6) gekoppelt werden zu können und das Herausgleiten des in der Vertiefung (60) aufgenommen Teils der Leitung (64) quer zur Erstreckungsrichtung der Vertiefung (60) verhindern oder erschweren zu können.

8. Verankerungsvorrichtung (2) nach Anspruch 6 und 7, **dadurch gekennzeichnet, dass** eine Schwenkachse des Kopplungsabschnittsscharniers (36), bezüglich welcher der Kopplungsabschnitt (38) relativ zu dem übrigen Teil (30) der Abdeckung (4) schwenkbar ist, parallel zu einer Schwenkachse des Abdeckungsscharniers (28) ist, bezüglich welcher die Abdeckung (4) relativ zur Basis (6) schwenkbar ist.

9. Verankerungsvorrichtung (2) nach Anspruch 7 oder 8, **dadurch gekennzeichnet, dass** die Basis (6) sowie die Abdeckung (4) integral ausgebildet sind und das Abdeckungsscharnier (28) sowie das Kopplungsabschnittsscharnier (36) als Filmscharniere ausgebildet sind.

10. Verankerungsvorrichtung (2) nach einem der Ansprüche 1 bis 9, **dadurch gekennzeichnet, dass** die Basis (6) eine Sensoraufnahme (22) aufweist.

11. System mit einer Verankerungsvorrichtung (2) nach einem der Ansprüche 1 bis 10 und einer Leitung (64).
